## Europäisches Patentamt
## European Patent Office
## Office européen des brevets

(19)

(11) Publication number: **0 136 093**
A2

(12)

# EUROPEAN PATENT APPLICATION

(21) Application number: **84305856.1**

(22) Date of filing: **28.08.84**

(51) Int. Cl.⁴: **A 61 K 37/02,** A 61 K 35/14, C 07 K 3/02, A 61 K 35/16

(30) Priority: **29.08.83 JP 158858/83**
**01.09.83 JP 161237/83**

(43) Date of publication of application: **03.04.85**
**Bulletin 85/14**

(84) Designated Contracting States: **DE FR GB**

(71) Applicant: **Sakagami, Yoshio, 3-16-19 Jugaoka,**
**Meguro-ku Tokyo (JP)**

(72) Inventor: **Sakagami, Yoshio, 3-16-19, Jugaoka,**
**Meguro-ku Tokyo (JP)**
Inventor: **Manube, Hobumi, 2-5-27, Inogashira,**
**Mitaka-shi Tokyo (JP)**

(74) Representative: **Bond, Bentley George et al, Haseltine**
**Lake & Co. 28 Southampton Buildings Chancery Lane,**
**London WC2A 1AT (GB)**

(54) **Anti-cancer factors.**

(57) Novel proteinaceaous substances which are active in inhibiting the proliferation of malignant tumour cells are disclosed. The substance may be derived from the blood, serum or organs of the living human body.

-1-

ANTI-CANCER FACTORS

This invention relates to novel proteinaceous substances which are active in inhibiting the proliferation of malignant tumour cells.

The compounds of the present invention may be derived from blood, serum or organs of the living human body and they show inhibitory action against the proliferation of various malignant tumour cells in in-vitro tests and are capable of prolonging the lives of animals with transplanted malignant tumour cells in in-vivo tests.

The compounds of the first, second and third aspects of the present invention are components of a substance named "cancer-cell proliferation inhibitory factor" by the inventors because of its peculiar chemical, physical and biological properties. This substance is isolated from human blood or sera and inhibits the growth of malignant tumour cells and markedly prolongs the lives of animals to which such malignant cells have been transplanted. Of special note

-2-

are its specific physiological activities; therefore, although it does not kill the malignant tumour cells, it significantly inhibits their growth, is nontoxic to humans and animals, and shows no appreciable adverse effects upon normal cells, thus making this new substance highly valuable as a medicine.

The novel proteinous cancer-cell proliferation inhibitory factor can be isolated from human blood or sera by a proper combination of known operations, such as salting out, extraction, adsorption, elution, dialysis, fractionation, precipitation, filtration, isoelectric precipitation, gel filtration, ion-exchange resin treatment and electrophoresis.

More specifically, crude powder of the cancer-cell proliferation inhibitory factor can be obtained by adding, to human blood or serum, ammonium sulphate, sodium chloride, acetone or alcohol to cause precipitation of the effective component, followed by purification through dialysis and drying.

Further purification can be achieved by column chromatography on ion-exchange resins, Sephadex, activated charcoal, silica gel or DEAE-cellulose, electrophoresis on polyacrylamide gel, sucrose or the like, isoelectric precipitation and ultra filtration alone or in combination. It is also possible to purify the cancer-cell proliferation inhibitory factor by adsorption on silica gel, bentonite, acid clay, activated charcoal or other suitable adsorbents.

The cancer-cell proliferation inhibitory factor thus isolated from human blood or serum has specific physiological activities as described above, and is useful as an anti-cancer agent.

Further studies have revealed that this

-3-

proteinaceous cancer-cell proliferation inhibitory factor is not a single substance  but is a mixture of several substances that can be separated from one another, each component being capable of inhibiting the growth of malignant tumour cells.   The individual components separated were named cancer-cell proliferation inhibitory factors A, B and C respectively.

Thus, according to a first aspect of the present invention, there is provided a glycoprotein, capable of being obtained from the human body, and designated A, characterised by:

(a)   being a white powder;

(b)   having a melting point in the range from 245°C to 249°C (with decomposition);

(c)   having a molecular weight of approximately 73,900 a.m.u. when measured by electrophoresis in 10% sodium dodecylsulphate solution;

(d)   being readily soluble in water, and insoluble in butanol, acetone, ethyl acetate, chloroform, benzene and hexane;

(e)   having an amino acid composition of 10.0% aspartic acid, 5.4% threonine, 3.8% serine, 16.0% glutamic acid, 4.2% proline, 1.4% glycine, 4.8% alanine, 2.6% cysteine, 5.1% valine, 0.8  methionine, 2.2% isoleucine, 9.6% leucine, 4.9% tyrosine, 5.7% phenylalanine, 11.4% lysine, 3.5% histidine and 5.2% arginine, each in error of ± 10% to the % values;

(f)   showing a positive response to the ninhydrine, anthrone, Molish and periodic acid reactions;

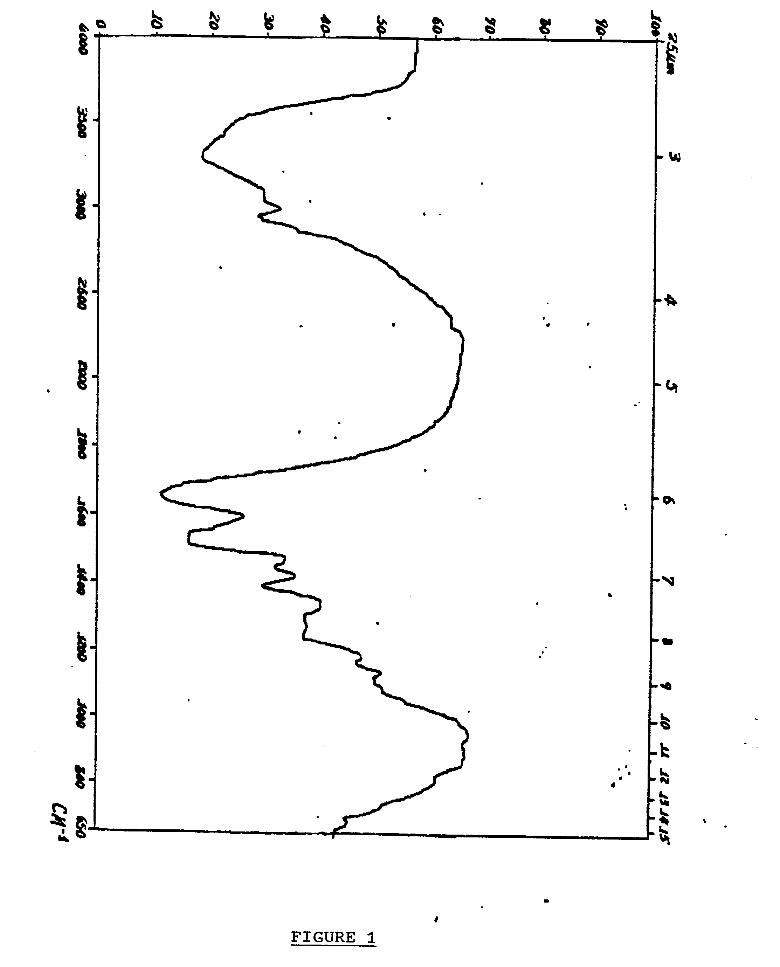

(g)   giving an infrared absorption  spectrum as shown in Figure 1 of the accompanying drawings (KBr pellet); and

-4-

(h)    exhibiting ultraviolet absorption maxima, in water, at 279nm at pH 3.0, at 278nm at pH 7.0 and at 277nm at pH 10.0.

According to a second aspect of the present invention there is provided a protein, capable of being obtained from the human body, designated B, characterised by:

(a)    being a white powder;

(b)    having a melting point in the range from 221°C to 225°C (with decomposition);

(c)    having a molecular weight of approximately 22,100 a.m.u. when measured by electrophoresis in 10% sodium doecylsulphate solution;

(d)    being readily soluble in water, and insoluble in acetone, ethyl acetate, benzene, hexane and chloroform;

(e)    having an amino acid composition of 16.0% aspartic acid, 5.2% threonine, 5.8% serine, 6.9% glutamic acid, 2.3% proline, 4.1% glycine, 5.9% alanine, 2.3% cysteine, 4.4% valine, 1.7% methionine, 4.2% isoleucine, 7.9% leucine, 4.0% tyrosine, 3.9% phenylalanine, 7.7% lysine, 1.6% histidine and 11.6% arginine, each in error of $\pm$ 10% to the % values;

(f)    showing a positive response to the ninhydrine reaction and a negative response to the anthrone, Molish and periodic acid reactions;

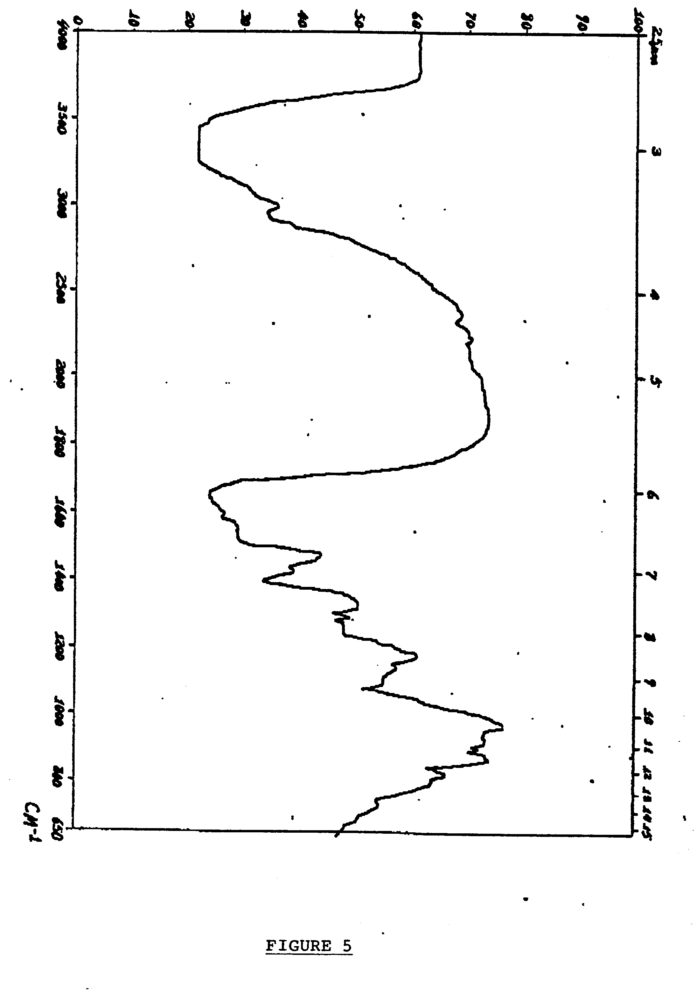

(g)    giving an infrared absorpotion spectrum as shown in Figure 5 of the accompanying drawings (KBr pellet); and

(h)    exhibiting ultraviolet absorption maxima in water at 283nm at pH 3.0, at 280nm atpH 7.0 and at 277nm at

0136093

-5-

pH 10.0.

According to a third aspect of the present invention there is provided a protein, capable of being obtained from the human body and designated C, characterised by:

(a)  being a white powder;

(b)  having a melting point in the range from 237°C to 240°C (with decomposition);

(c)  having a molecular weight of approximately 14,000 a.m.u. when measured by electrophoresis in 10% sodium dodecylsulphate solution;

(d)  being readily soluble in water, and insoluble in acetone, ethyl acetate, benzene, hexane and chloroform;

(e)  having an amino acid compostion of 18.0% aspartic acid, 5.4% threonine, 6.4% serine, 5.2% glutamic acid, 1.7% proline, 5.1% glycine, 6.4% alanine, 2.1% cysteine, 4.3% valine, 1.9% methionine, 4.5 isoleucine, 6.8% leucine, 3.6% tyrosine, 3.0% phenylalanine, 6.0% lysine, 1.1% histidine and 11.6% arginine, each in error of ± 10% to the % values;

(f)  showing a positive response to the ninhydrine reaction and a negative response to the anthrone, Molish and periodic acid reactions;

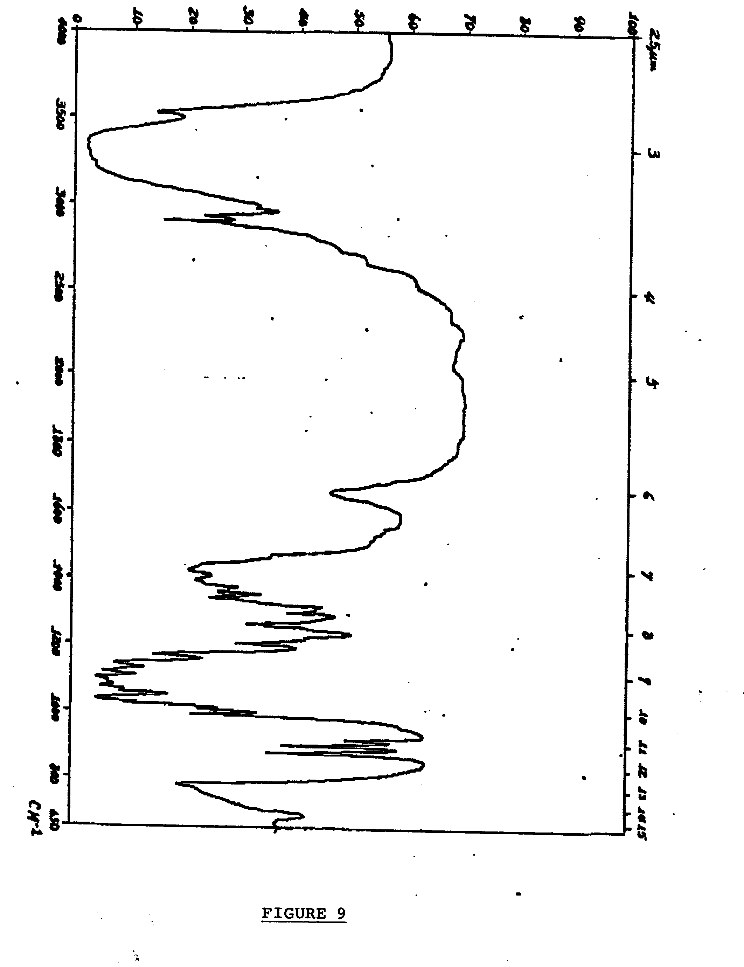

(g)  giving an infrared absorption spectrum as  shown in Figure 9 of the accompanying drawings (KBr pellet); and

(h)  exhibiting ultraviolet absorption maxima in water at 278nm at pH 3.0, at 290nm at 7.0, and at 277nm at pH 10.0.

-6-

According to a fourth aspect of the present invention, there is provided a composition which comprises one or more of the proteins A, B and C as described above.

These factors A, B and C, can be separated from one another if the salting-out operation mentioned above is performed at different degrees of alcohol saturation, for example, 40%, 50% or 60% saturation, depending on the molecular weight of the factor, each precipitate, after confirmation of its purity by adsorption and elution with molecular sieves, is carefully fractionated by means of column chromatography.

Cancer-cell Proliferation Inhibitory Factor A

Appearance      : White powder

Melting point   : 245 - 259°C ( dec )

Structure       : Glycoprotein

Molecular weight: Approximately 73,900 ( when measured by electrophoresis in 10% sodium dodecyl sulphate solution )

Solubility      : Readily soluble in water; insoluble in butanol, acetone, ethyl acetate, chlorform, benzene and hexane

Amino acid composition:

10.0% aspartic acid, 5.4% threonine, 3.8% serine, 16.0% glutamic acid, 4.2% proline, 1.4% glycine, 4.8% alanine, 2.6% cysteine, 5.1% valine, 0.8% methionine, 2.2% isoleucine, 9.6% leucine, 4.9% tyrosine, 5.7% phenylalanine, 11.4% lysine, 3.5% histidine and 5.2% arginine

Colour reaction : Positive response to the ninhydrine, anthrone, Molish and periodic acid reactions

Infrared absorption spectrum: Refer to Fig. 1 ( measured on KBr pellet ).

Ultraviolet absorption spectra: Refer to Figs. 2, 3 and 4.

Absorption maximum at 279nm in water of pH 3.0 ( Fig. 2 ) Absorption maximum at 278nm in water .of pH 7.0 ( Fig. 3 ) Absorption maximum at 277nm in water of pH 10.0 ( Fig. 4 )

Cancer-cell Proliferation Inhibitory Factor B

| | |
|---|---|
| Appearance | : White powder |
| Melting point | : 221 - 225 °C ( dec ) |
| Structure | : Protein |
| Molecular weight: | Approximately 22,100 ( when measured by electrophoresis in 10% sodium dodecyl sulphate solution ) |
| Solubility | : Readily soluble in water; insoluble in acetone, ethyl acetate, chlorform, benzene and hexane |

Amino acid composition:

16.0% aspartic acid, 5.2% threonine, 5.8% serine, 6.9% glutamic acid, 2.3% proline, 4.1% glycine, 5.9% alanine, 2.3% cysteine, 4.4% valine, 1.7% methio-

-9-

nine, 4.2% isoleucine, 7.9% leucine, 4.0% tyrosine, 3.9% phenylalanine, 7.7% lysine, 1.6% histidine and 11.6% arginine

Colour reaction : Positive response to the ninhydrine reaction, and negative response to the anthrone, Molish and periodic acid reactions

Infrared absorption spectrum: Refer to Fig. 5 ( measured on KBr pellet ).

Ultraviolet absorption spectra:

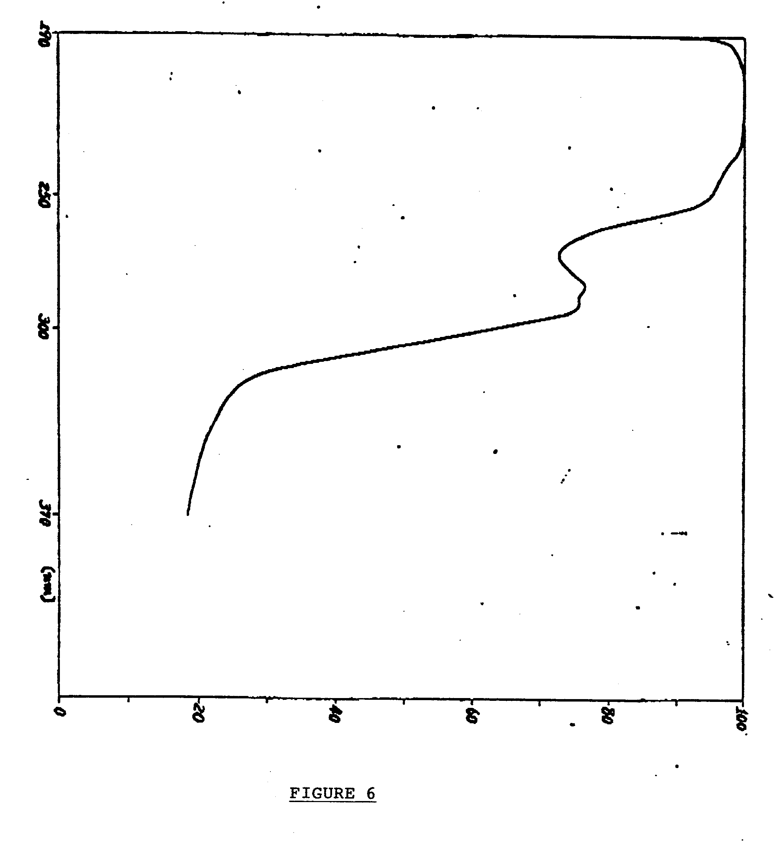

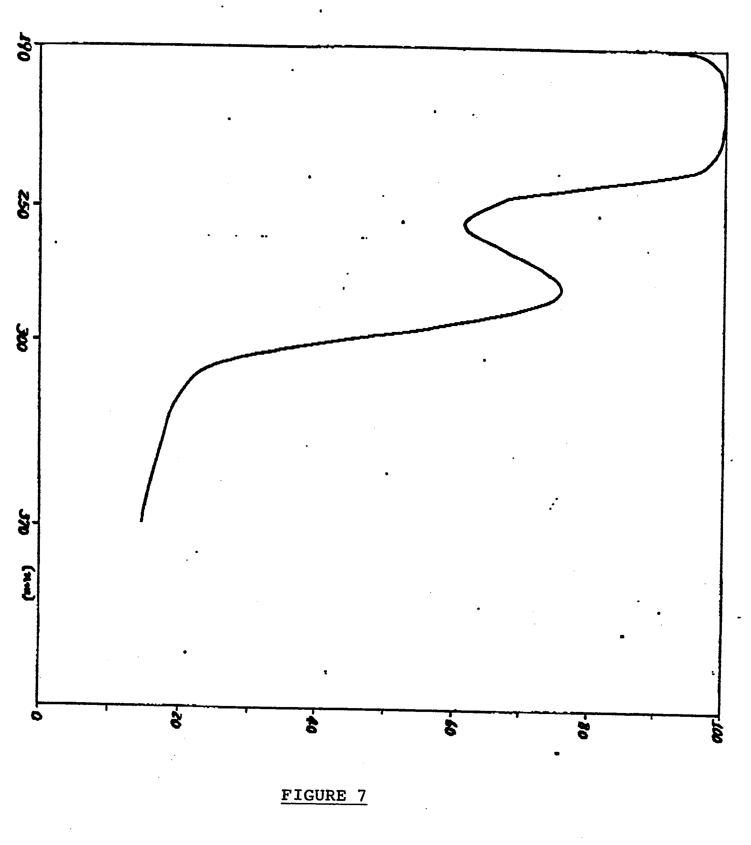

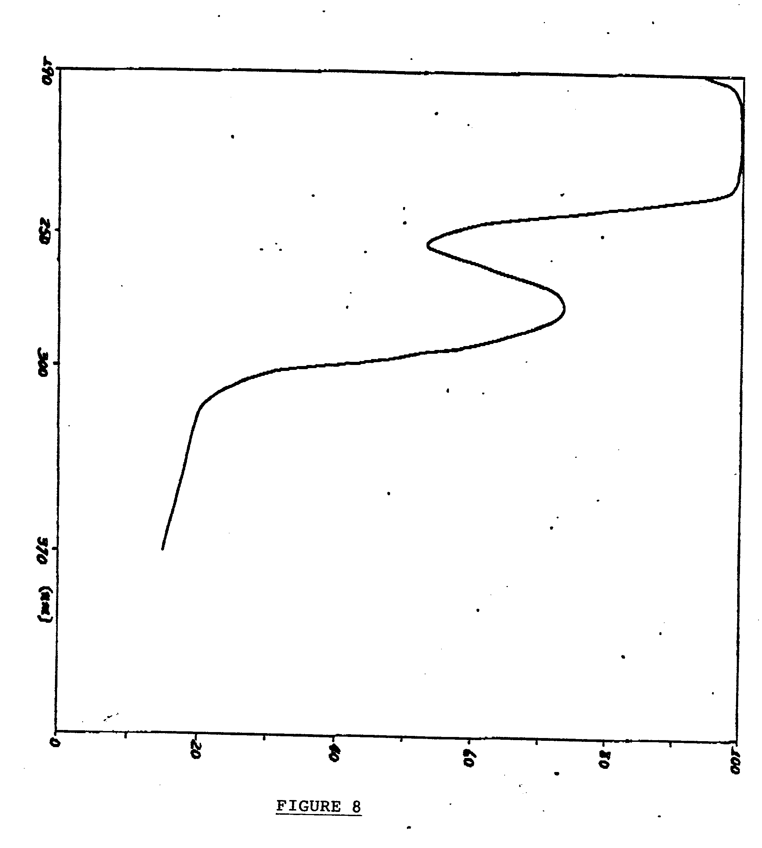

Absorption maxima at 283nm and 290nm in water of pH 3.0 ( Fig. 6 ) Absorption maximum at 280nm in water of pH 7.0 ( Fig. 7 ) Absorption maximum at 277nm in water of pH 10.0 ( Fig. 8 )

Cancer-cell Proliferation Inhibitory Factor C

Appearance : White powder

Melting point : 237 - 240°C ( dec )

Structure : Protein

Molecular weight: Approximately 14,000 ( when measured by electrophoresis in 10% sodium dodecyl sulphate solution )

Solubility : Readily soluble in water; insoluble

-10-

in acetone, ethyl acetate, chlorform,
benzene and hexane

Amino acid composition:

18.0% aspartic acid, 5.4% threonine,
6.4% serine, 5.2% glutamic acid, 1.7%
proline, 5.1% glycine, 6.4% alanine,
2.1% cysteine, 4.3% valine, 1.9%
methionine, 4.5% isoleucine, 6.8% leucine, 3.6% tyrosine, 3.0% phenylalanine, 6.0% lysine, 1.1% histidine
and 11.6% arginine

Colour reaction : Positive response to the ninhydrine
reaction, and negative response to the
anthrone, Molish and periodic acid
reactions

Infrared absorption spectrum: Refer to Fig. 9 ( measured
on KBr pellet ).

Ultraviolet absorption spectra:

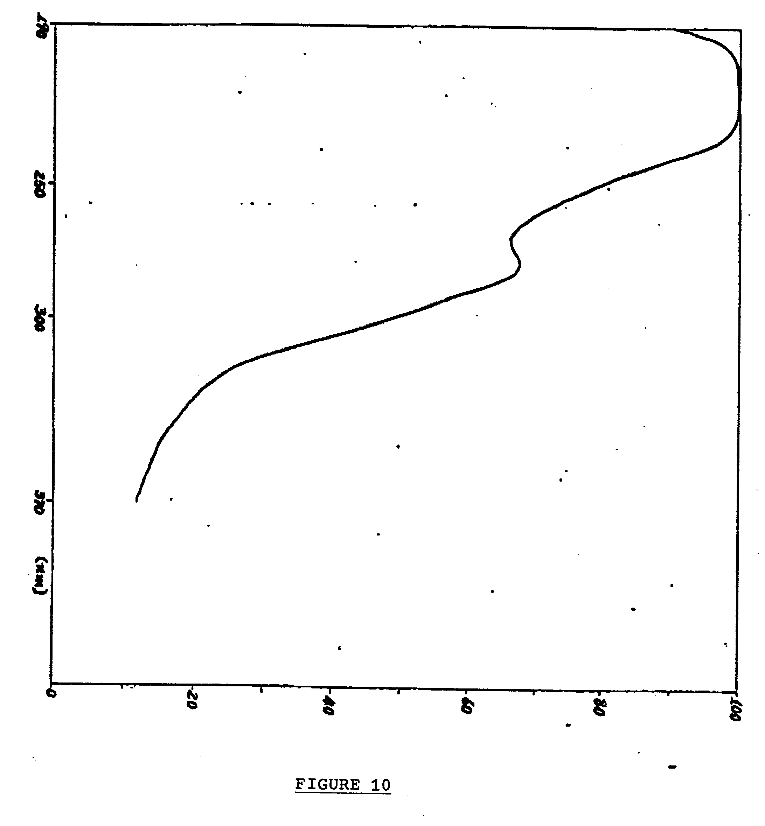

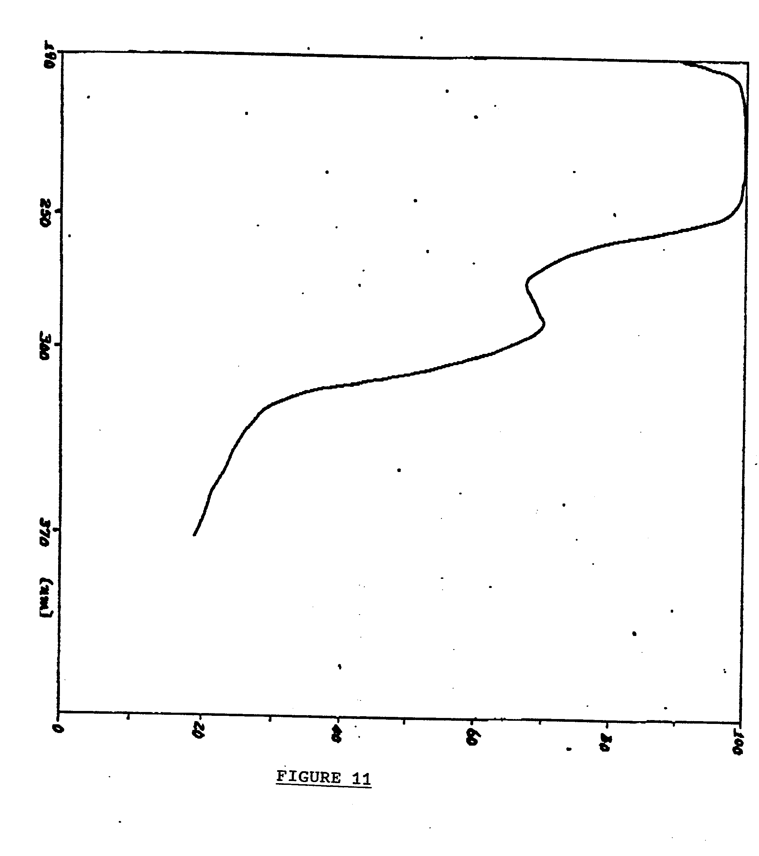

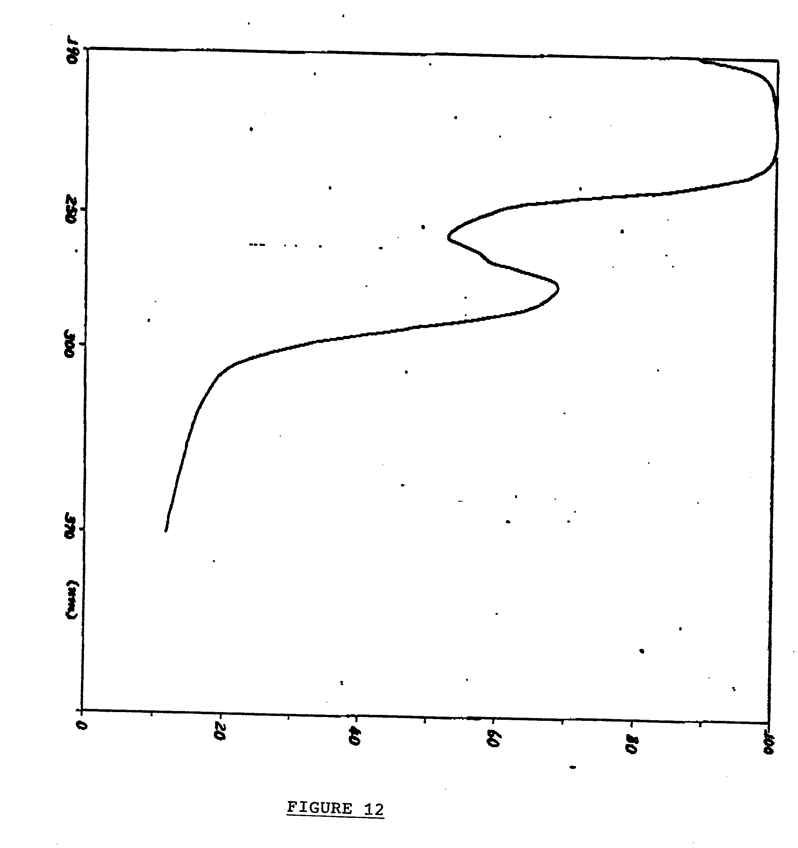

Absorption maximum at 278nm in water
of pH 3.0 ( Fig. 10 )
Absorption maximum at 290nm in water
of pH 7.0 ( Fig. 11 )
Absorption maximum at 277nm in water
of pH 10.0 ( Fig. 12 )

-11-

When subjected to electrophoresis on 7% polyacrylamide gel, factors A, B and C each migrate from the origin point at pH 8.0, 9.5 and 10.0, respectively, forming a single band.

It is therefore possible to separate them from one another by using this method in place of column chromatography. The factors thus purified, as well as the crude powder obtained in preceding purification steps, can be used as medicines to inhibit the proliferation of various malignant tumour cells.

They may also be used in a variety of dosage forms, such as parenteral injections.

The following examples will further illustrate the first, second, third and fourth aspects of the present invention.

Example 1

Two litres of acetone were added to one litre of human serum at -20°C, and the precipitate collected by centrifugation, was dissolved in as little distilled water as possible. Fractional precipitation by addition of ethanol to a concentration of from 45 to 50% gave 8.6g of solid.

Five grams of this precipitate was adsorbed on 20ml Sephadex G-200 at pH 7.3, which was follwed by elution with 30ml of 0.05M Tris-HCl buffer solution ( pH: 7.3 ), affording 0.9g of crude glycoprotein A.

This was then adsorbed on DEAE-cellulose ( Whatman ),

-12-

and eluted with 40ml of sodium chloride solution ( concentration gradient: 0 to 0.5M ) to give seven fractions roughly separated. The fifth fraction was dialyzed with 0.02M Tris buffer solution, desalted and freeze-dried, yielding 23.7mg of crude product.

This crude product ( 50mg ) was again adsorbed on DEAE-cellulose ( Whatman ), eluted with 0.1M sodium chloride solution at pH gradient from 8.0 to 5.0 to give five fractions, and the second fraction was desalted and freeze-dried to afford 7.3mg of glycoprotein A.

This was further subjected to electrophoresis on 7% polyacrylamide gel for four hours, and the second band which showed a positive response to the ninhydrine test was eluted with 0.02M Tris buffer solution, followed by dialysis and drying. The resulting glycoprotein A (0.6mg) showed a single band in electrophoresis.

Tissue culture tests showed that the rate of inhibition against the growth of leukemia L1210 cells by this substance is 99% and 61.5% at concentrations of 0.01 mg/ml and 0.001 mg/ml, respectively.

Example 2

The fifth fraction obtained, in the same manner as Example 1, from the first chromatogrphy on DEAE-cellulose ( 31.2mg ) was subjected to electrophoresis on polyacryl-

-13-

amide gel at pH 9.5 to isolate the effective component as a single substance. Dialysis followed by freeze drying gave 0.6mg of purified glycoprotein A as white powder.

Example 3

Eight litres of human serum was treated with ethanol ( 60% of the serum ), the precipitate was removed, and ethanol was added to the supernatant to a concentration of 70%. The resulting precipitate was adsorbed on 40ml Sephadex G-50 at pH 7.3, and eluted with 50ml of Tris-HCl buffer solution ( pH: 7.3 ), affording 5.8g of crude protein B.

This was then adsorbed on DEAE-cellulose ( Whatman ), and eluted with 50ml of Tris-HCl buffer solution ( pH 7.0; ion strength gradient    0.02 to 0.05M ) to give six fractions.

Dialysis, desalting and freeze drying of the first fraction gave 25.4mg of crude product.

This was further adsorbed on DEAE-cellulose ( Whatman ( eluted with 0.1M sodium chloride solution ( pH gradient: 8.0 to 5.0 ) to give six fractions, and the first fraction was dialyzed, desalted and freeze-dried, yielding 4.2mg of purified protein B as white powder.

This substance showed a single band in electrophoresis. Tissue culture tests showed that addition of this substance

-14-

( 400 mcg/ml ) declined the angle of growth curve for leukemia L1210 cells by about 20 degrees.

Example 4

Two litres of human serum was treated with ethanol in the same manner as Example 3, the precipitate was adsorbed on DEAE-cellulose ( Whatman ), and eluted with 0.1M sodium chloride solution ( pH gradient: 8.0 to 5.0 ) to give six fractions. The first fraction was desalted by dialysis, freeze-dried, and subjected to electrophoresis on 7% polyacrylamide gel to isolate the effective component as a single substance. Protein B ( 85mcg ) was thus obtained as white powder after dialysis and freeze drying.

Tissue culture tests similar to Example 3 showed that this substance is also effective in declining the angle of growth curve for leukemia L1210 cells.

Example 5

Eight litres of human serum was treated with ethanol ( 70% of the serum ), the precipitate was removed, and the supernatant was concentrated under reduced pressure and freeze-dried. This solid was adsorbed on 40ml Sephadex G-50 at pH 7.3, and eluted with 30ml of 0.05M Tris-HCl buffer solution ( pH: 7.3 ), affording about 1.0g of crude Protein C.

This was then adsorbed on DEAE-cellulose ( Whatman ), and eluted with 50ml of Tris-HCl buffer solution ( pH 7.0;

-15-

ion strength gradient: 0.02 to 0.05M ) to give three fractions.

Dialysis, desalting and freeze drying of the second fraction gave 14.5mg of crude product.

This was further adsorbed on DEAE-cellulose ( Whatman ), eluted with 0.1M sodium chloride solution ( pH gradient: 8.0 to 5.0 ) to give six fractions, and the fourth fraction was desalted by dialysis and freeze-dried, yielding 1.8mg of purified Protein C as white powder.

This substance showed a single band in electrophoresis. Tissue culture tests showed that addition of this substance ( 500 mcg/ml ) declined the angle of growth curve for leukemia L1210 cells by about 10 degrees.

Example 6

Two litres of human serum was treated in the same manner as Example 5, the freeze-dried product obtained from the supernatant was adsorbed on DEAE-cellulose ( Whatman ), and eluted with 0.1M sodium chloride solution ( pH gradient: 8.0 to 5.0 ) to give six fractions. The fourth fraction was desalted by dialysis, freeze-dried, and subjected to electrophoresis on polyacrylamide gel to isolate the effective component as a single substance. Protein C ( 184mcg ) was thus obtained as white powder after dialysis and freeze drying.

Tissue culture tests similar to Example 5 showed that

-16-

this substance is also effective in declining the angle of growth curve for leukemia L1210 cells.

Example 7

Tissue culture tests were conducted to determine the effect to inhibit the proliferation of various malignant tumor cells, for the glycprotein A samples obtained in Examples 1 and 2 ( at 500 mcg/ml concentration ).

The inhibition rate was 32.6% for leukemia L5178 cells, 21.3% for sarcoma S180 cells, and 32.3% for Ehrlich's carcinoma cells, but no such inhibitory effect was observed for the primary tissue-cultured cells of rats liver.  Subsequent staining revealed that all the tested malignant tumor cells remined alive.

Example 8

An animal test was carried out for glyprotein A obtained in Example 1 using four groups of male BDF mice ( weighing 20.0g ), each group consisting of five head.

In the first group, 0.1mg of T.D.F.H.A was abdominally administered to each mouse 24 hours after abdominal transplantation of leukemia L1210 cells ( $1 \times 10^6$ ).  Similarly, 0.01mg and 0.001mg of the sample was administered in the second and third groups.

The average life time was 9.7 days for the control group, 20.0 days for the first group, 15.7 days for the second and 12.3 days for the third, the T/c value being 207,

-17-

162 and 128 for the first, second and third groups, respectively.

Example 9

Tissue culture tests showed that protein B obtained in Example 3 ( at 500 mcg/ml concentration ) declined the angle of growth curves for leukemia L5178Y cells, sarcoma S180 cells and Ehrlich's carcinoma cells by about 15 degrees, 14 degrees and 5 degrees, respectively.

On the other hand, no such inhibitory effect was observed for the primary tissue-cultured cells of rat liver.

Subsequent staining demonstrated that all the malignant tumour cells tested remained alive after the test.

Example 10

Tissue culture tests showed that protein C obtained in Example 5 and 6 ( at 500 mcg/ml concentration ) declined the angle of growth curves for leukemia L5178Y cells, sarcoma S180 cells and Ehrlich's carcinoma cells by 1 degree, about 22 degrees and 1 degree, respectively.

On the other hand, no such inhibitory effect was observed for the primary tissue-cultured cells of rat liver.

Subsequent staining demonstrated that all the malignant tumour cells tested remained alive after the test.

-18-

The compounds of fifth and sixth aspects of the present invention are compounds of a substance named "cancer-cell killing and proliferation inhibiting factor" by the inventors because of its peculiar chemical, physical and biological properties. This substance is isolated from human blood, and inhibits the growth of malignant tumour cells and markedly prolongs the lives of animals to which such malignant cells have been transplanted. Of special note are its specific physiological activities; thus, it has prominent actions to kill malignant tumour cells or to inhibit their growth, it is nontoxic to humans and animals, and shows no appreciable adverse effects upon normal cells, making this new substance highly valuable as a medicine.

The novel proteinaceous cancer-cell killing and proliferation inhibiting factor can be isolated from human blood by a proper combination of known operations, such as salting out, extraction, adsorption, elution, dialysis, fractionation, precipitation, filtration, isoelectric precipitation, gel filtration, ion-exchange resin treatment and electrophoresis. More specifically, crude powder of the cancer-cell killing and proliferation factor can be obtained by adding, to human blood or clot extracts, ammonium sulphate, sodium chloride, acetone, alcohol or the like to cause precipitation of the effective component, followed by purification through dialysis and drying.

Further purification can be achieved by column chromatography on ion-exchange resins, Sephadex, activated charcoal, silica gel, cellulose or DEAE-cellulose, electrophoresis or polyacrylamide gel, sucrose or the like, isoelectric precipitation and ultrafiltration, alone or in combination.

It is also possible to purify the cancer-cell killing and proliferation factor by adsorption on silica gel, bentonite, acid clay, activated charcoal or other suitable adsorbents.

The cancer-cell killing and proliferation factor thus isolated from human blood or clot has specific physiological activities as described above, and is useful as an anti-cancer agent.

Further studies have revealed that this proteinaceous cancer-cell killing and proliferation inhibiting factor is not a single substance but is a mixture of several substances that can be separated from one another, each component being capable of inhibiting the growth of malignant tumour cells. The individual components separated are named cancer-cell killing and proliferation inhibiting factors D and E respectively.

Thus, according to a fifth aspect of the present invention, there is provided a protein, capable of being obtained from the human body, and designated D, characterised by:

(a)   being a white powder;

(b)   melting in the range from 218°C to 221°C (with decomposition);

(c)   having a molecular weight of approximately 40,000 a.m.u. when measured by electrophoresis in 10% sodium dodecylsulphate solution;

(d)   being readily soluble in water, and insoluble in butanol, acetone, ethyl acetate, chloroform, benzene and hexane;

(e)   having an amino acid composition of 8.9% aspartic acid, 5.7% threonine, 4.3% serine, 6.3% glutamic acid, 3.8% proline, 5.8% glycine, 5.8% alanine, 0.6% cysteine, 5.2% valine, 0.5% methionine, 2.2% isoleucine, 4.7% leucine, 3.0%

-20-

tyrosine, 2.6% phenylalanine, 2.4% lysine, 1.3% histidine and 4.4% **arginine,** each in error of $\pm$ 10% to the % values;

(f) showing a positive respone to the ninhydrine reaction and a negative response to the anthrone, Molish and periodic acid reactions;

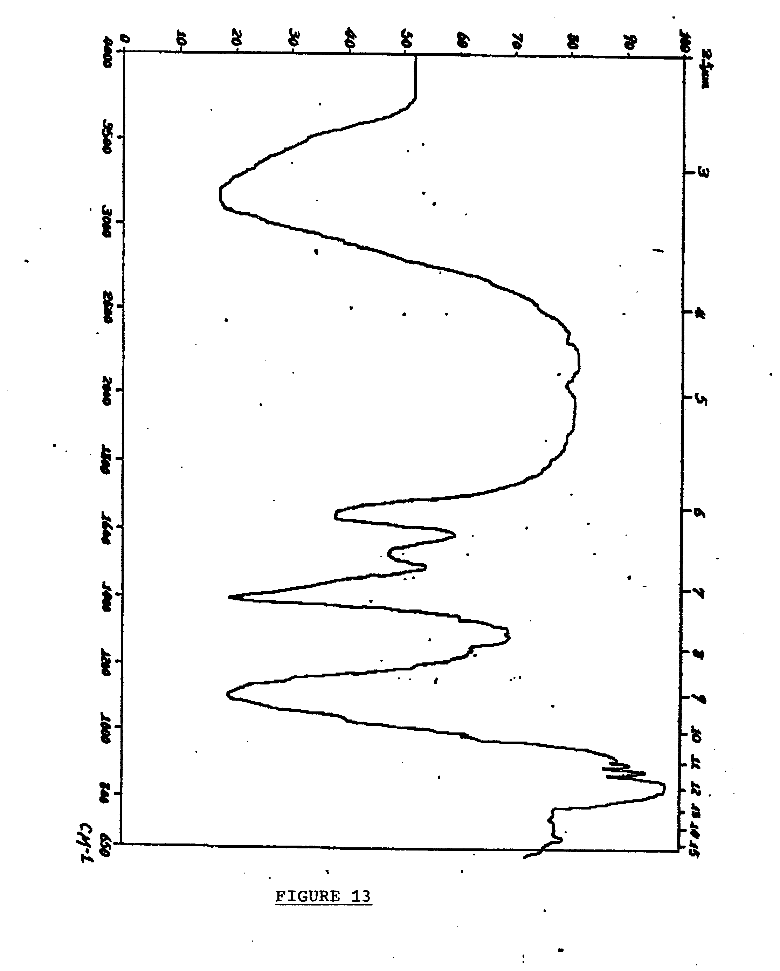

(g) giving an infrared absorption spectrum as shown in Figure 13 of the accompanying drawings (KBr pellet); and

(h) exhibiting ultraviolet absorption in water at 275nm, 282.5nm and 291.5nm at pH 5.0; at 275nm, 282.5nm and 291.5nm at pH 7.0; and at 282nm and 291.5nm at pH 10.0.

According to a sixth aspect of the present invention there is provided a protein, capable of being obtained from the human body and designated E, characterised by:

(a) being a white powder;

(b) having a melting point in the range from 231°C to 233°C (with decomposition);

(c) having a moleuclar weight of approximately 44,000 a.m.u. when measured by electrophoresis in 10% sodium dodecylsulphate solution;

(d) being readily soluble in water, and insoluble in acetone, ethyl acetate, chloroform, benzene and hexane;

(e) having an amino acid composition of 9.2% aspartic acid, 5.7% threonine, 4.3% serine, 8.6% glutamic acid, 4.0% proline, 4.7% glycine, 5.6% alanine, 1.0% cysteine, 5.2% valine, 0.6% methionine, 2.2% isoleucine, 6.0% leucine, 3.4% tyrosine, 3.1% phenylalanine, 4.4% lysine, 1.9% histidine and 4.7% arginine,
each in error of ± 10% to the % values;

(f) showing a positive response to the ninhydrine reaction and a negative response to the anthrone, Molish and periodic acid reactions;

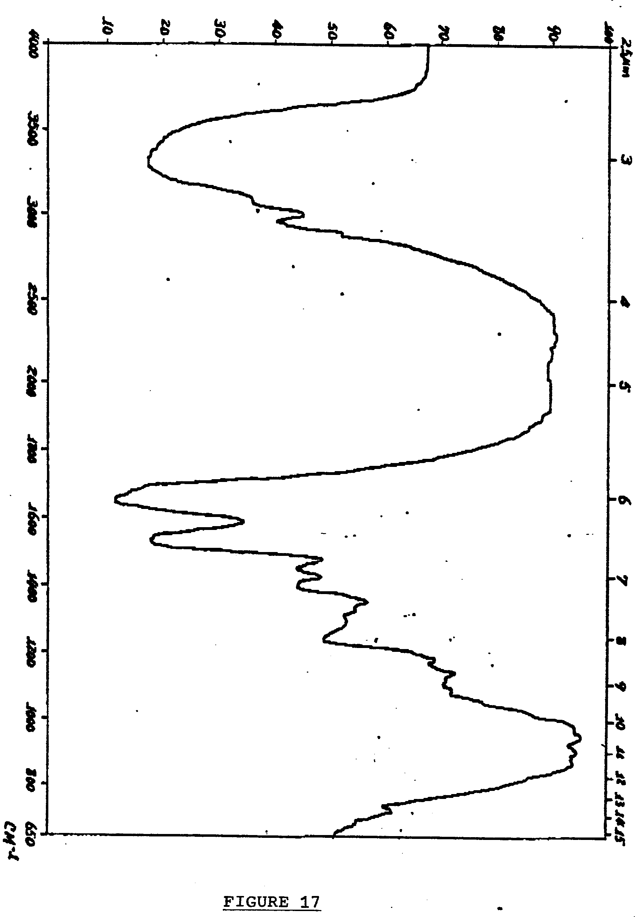

(g) giving an infrared absorption spectrum as shown in Figure 17 of the accompanying drawings (KBr pellet); and

(h) exhibiting ultraviolet absorption maxima in water at 280nm (with a shoulder at 249nm) at pH 5.0, at 280nm (with a shoulder at 291nm) at pH 7.0, and at 281nm (with a shoulder at 293nm) at pH 10.0.

Further, according to a seventh aspect of the present invention, there is provided a composition which comprises the proteins D and E as described above.

These factors, D, E, can be separated from one another if the salting-out operation mentioned above is performed at different degrees of salt saturation depending on the molecular weight of the factor, and each precipiate, after confirmation of its purity by adsorption and elution with molecular sieves, is carefully fractionated by means of column chromatography.

Cancer-cell Killing and Proliferation Inhibiting Factor D

| | |
|---|---|
| Appearance | : White powder |
| Melting point | : 218 - 221°C ( dec ) |
| Structure | : Protein |
| Molecular weight | : Approximately 40,000 (when measured by electrophoresis in 10% sodium dodecylsulphate solution) |
| Solubility | : Readily soluble in water; insoluble in butanol, acetone, ethyl acetate, |

-22-

chlorform, benzene and hexane

Amino acid composition:

8.9% aspartic acid, 5.7% threonine, 4.3% serine, 6.3% glutamic acid, 3.8% proline, 5.8% glycine, 5.8% alanine, 0.6% cysteine, 5.2% valine, 0.5% methionine, 2.2% isoleucine, 4.7% leucine, 3.0% tyrosine, 2.6% phenylalanine, 2.4% lysine, 1.3% histidine and 4.4% arginine

Colour reaction : Positive response to the ninhydrine reaction, and negative response to the anthrone, Molish and periodic acid reactions

Infrared absorption spectrum: Refer to Fig. 13 ( measured on KBr pellet ).

Ultraviolet absorption spectra:

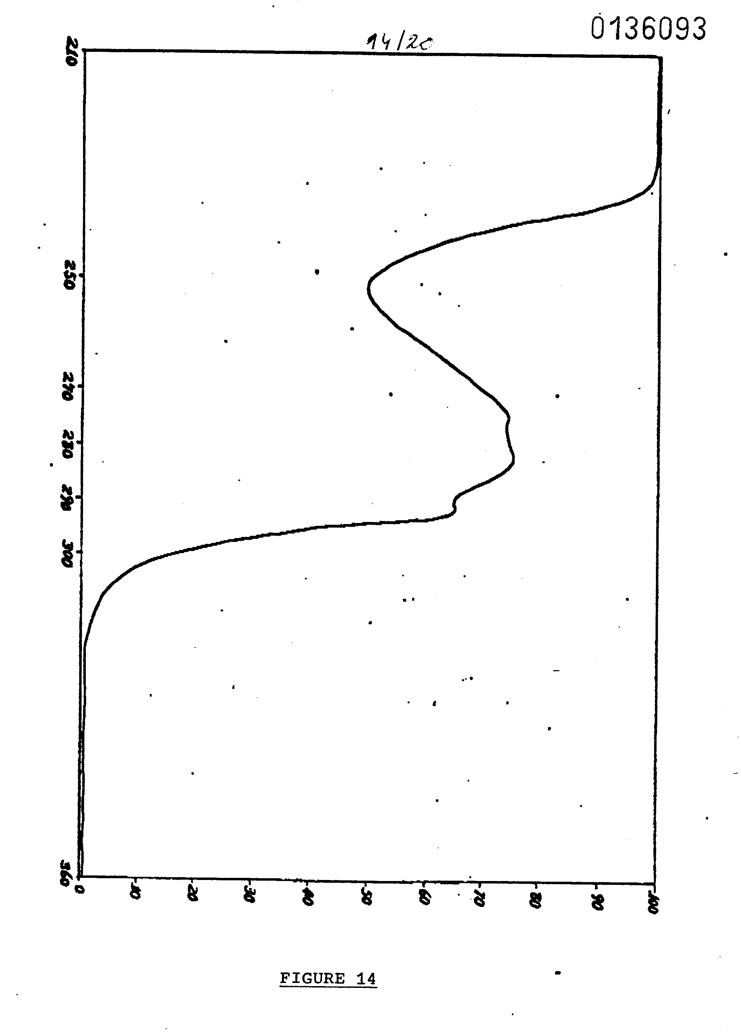

Absorption maxima at 275nm, 282.5nm and 291.5nm in water of pH 5.0 ( Fig. 14 )

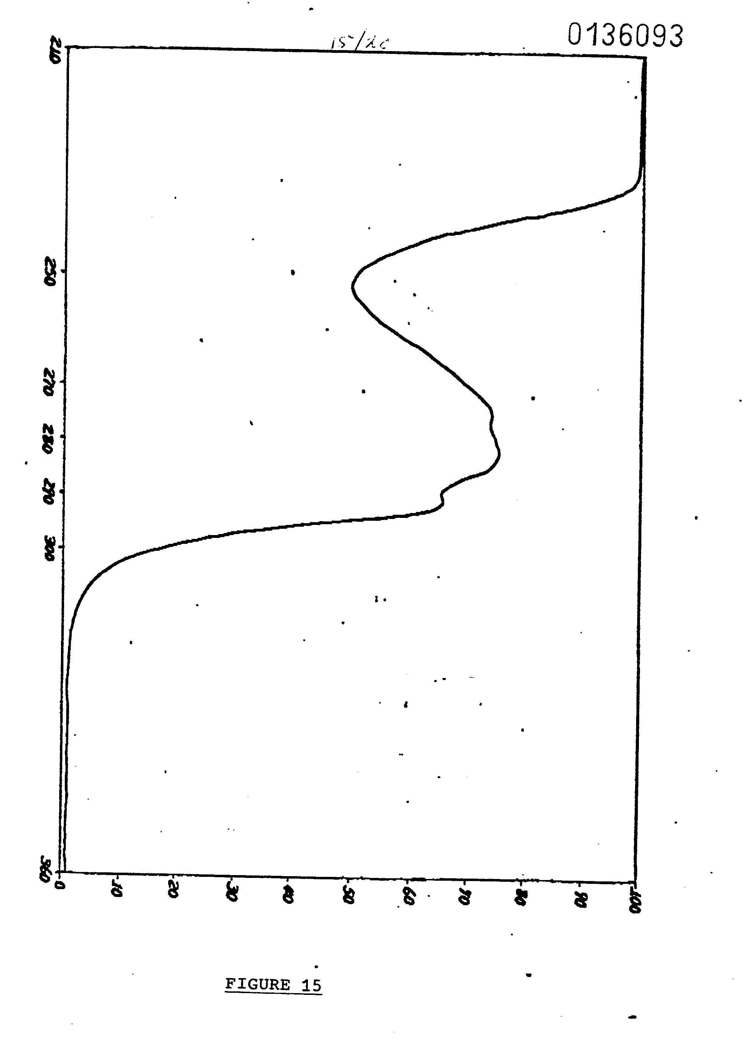

Absorption maxima at 275nm, 282.5nm and 291.5nm in water of pH 7.0 ( Fig. 15 )

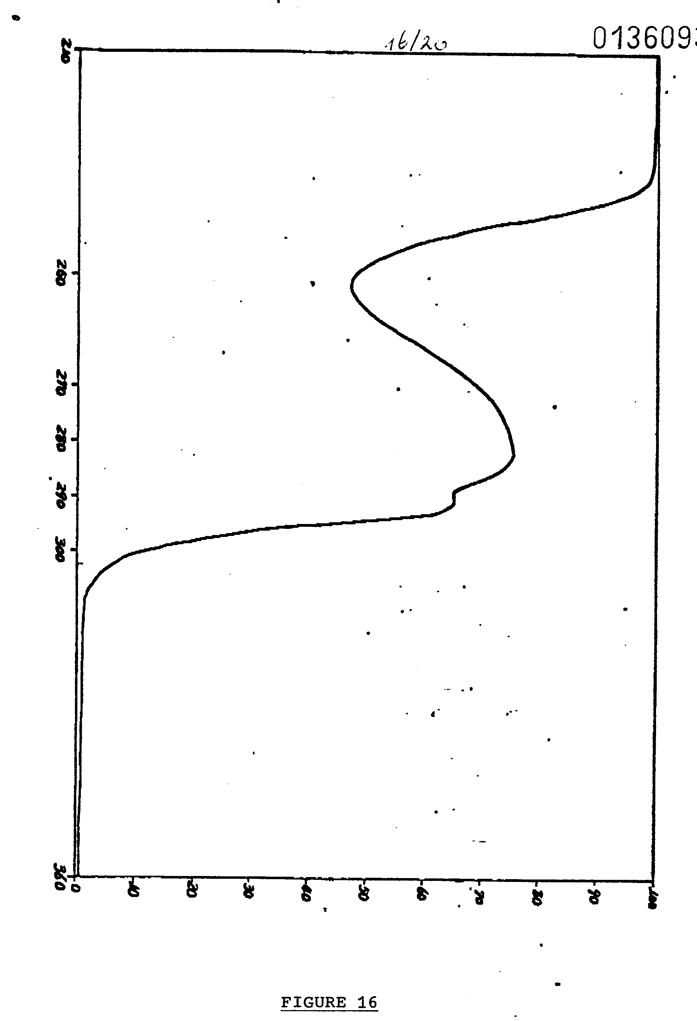

Absorption maxima at 282nm and 291.5nm in water of pH 10.0 ( Fig. 16 )

Cancer-Cell Killing and Proliferation Inhibiting Factor E

Appearance        : White powder Melting point     : 231 - 233°C ( dec ) Structure         : Protein Molecular weight: Approximately 44,000 ( when measured by electrophoresis in 10% sodium dodecyl sulphate solution )

Solubility        ; Readily soluble in water; insoluble in butanol, acetone, ethyl acetate, chlorform, benzene and hexane

Amino acid composition:

9.2% aspartic acid, 5.7% threonine, 4.3% serine, 8.6% glutamic acid, 4.0% proline, 4.7% glycine, 5.6% alanine, 1.0% cysteine, 5.2% valine, 0.6% methionine, 2.2% isoleucine, 6.0% leucine, 3.4% tyrosine, 3.1% phenylalanine, 4.4% lysine, 1.9% histidine and 4.7% arginine

Colour reaction   : Positive response to the ninhydrine reaction, and negative response to the anthrone, Molish and periodic acid reactions

Infrared absorption spectrum: Refer to Fig. 17 ( measured

-24-

on KBr pellet ).

Ultraviolet absorption spectra:

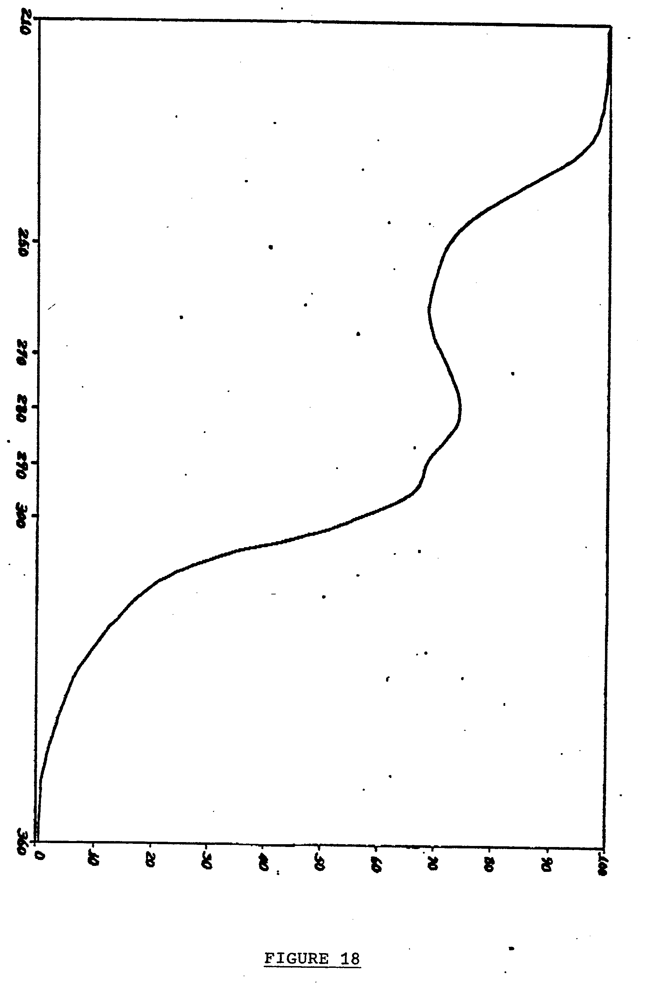

Absorption maximum at 280nm with a shoulder at 294nm in water of pH 5.0 ( Fig. 18 )

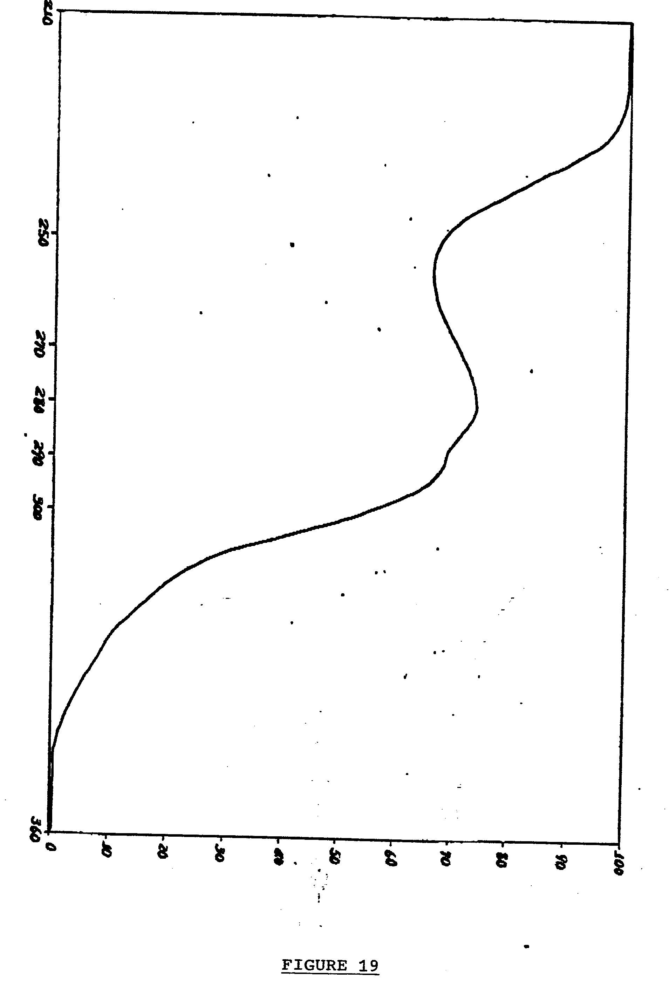

Absorption maximum at 280nm with a shoulder at 291nm in water of pH 7.0 ( Fig. 19 )

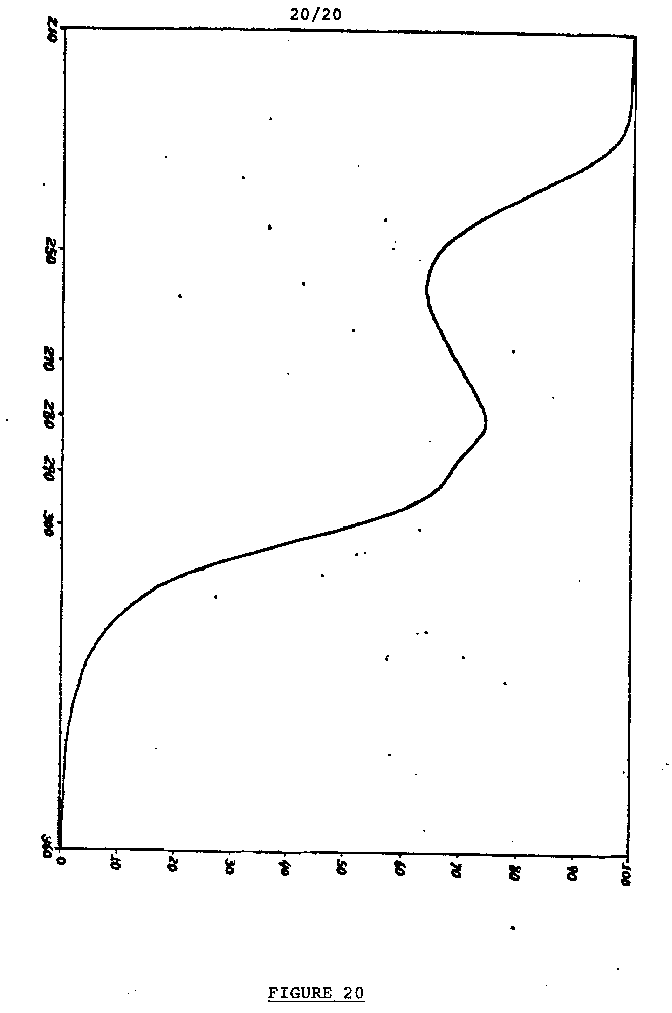

Absorption maximum at 281nm with a shoulder at 293nm in water of pH 10.0 ( Fig. 20 )

When subjected to electrophoresis on 7% polyacrylamide gel, Proteins A and B migrate from the origin point at pH 6.0, each forming a single band.

It is therefore possible to separate them from one another by using this method in place of column chromatography or ultrafiltration. The factors thus purified, as well as crude powder obtained in preceding purification steps, can be used as medicines to inhibit the proliferation of various malignant tumor cells.

They may also be used in a variety of dosage forms, such as parenteral injections.

The following examples will further illustrate the fifth, sixth and seventh aspects of the present invention.

-25-

Example 11

Water-soluble component ( 8.4g ) obtained from human clot was dissolved in water, and ethanol was added to the solution to a saturation degree of 65 to 70% to cause precipitation, giving about 700mg of the crude cancer-cell killing and proliferation inhibitory factor.

Tissue culture tests on leukemia L1210 cells using this substance showed that the relative survival rate ( based on a control culture sample ) after five days was 64%, 79% and 90% at concentrations of 1 mg/ml, 0.1 mg/ml and 0.01 mg/ml, respectively, the realtive activity to inhibit the cell growth being 36%, 21% and 10%.

Example 12

The substance obtained in the same way as Example 11 ( 350mg ) was adsorbed on 30ml of Sephadex G-100 at pH 7.0, and eluted with 30ml of 0.05M Tris-HCl buffer solution, affording about 60mg of the crude cancer-cell killing and proliferation inhibitory factor
Similar tissue culture tests as Example 11 showed that the relative survival rate of leukemia L1210 cells was 34 % after five days at a concentration of 0.1 mg/ml, the relative activity to inhibit the cell growth being 64%.

Example 13

Water-soluble component ( 2.1g ) obtained from human clot was dissolved in water, and ammonium sulphate was added to the solution to a saturation degree of 80% to cause pre-

-26-

cipitation, giving about 60mg of the crude cancer-cell killing and proliferation inhibitory factor.

Tissue culture tests on sarcoma 180 cells using this substance showed that the relative survival rate ( based on a control culture sample ) after five days was 49%, 61% and 73% at concentrations of 1 mg/ml, 0.1 mg/ml and 0.01 mg/ml, respectively, the relative activity to inhibit the cell growth being 51%, 39% and 27%.

Example 14

Four grams of the crude cancer-cell killing and proliferation inhibitory factor obtained in the same manner as Example 12 were adsorbed on DEAE-cellulose (Whatman), and eluted with Tris-HCl buffer solution ( pH: 7.0 ) at an ion strength gradient of from 0.03M to 0.05M to give five fractions.

The third fraction was subdivided into four fractions in the same way as above to give four fractions, the second fraction of which was dialyzed with 0.02M Tris buffer solution, desalted and freeze-dried, affording 9.1mg of Protein E as white powder.

From the fourth fraction ( of the second DEAE-cellulose chromatography ) was obtained 14.1mg of Protein D as white powder after dialysis with 0.02M Tris buffer solution, followed by freeze drying.

The Proteins D and E isolated above each exhibited a single band when subjected to electrophoresis.

A tissue culture test on leukemia L1210 cells using the Protein D showed that the relative survival rate ( based on a control culture sample ) after five days was 15%, the relative activity to inhibit the cell growth being 85%.

A similar test using the cancer-cell killing and proliferation inhibitory factor showed a relative survival rate of 53% and a relative activity of 47% after five days at the same concentation.

Example 15

The fourth fraction ( 70mg ), obtained from the second DEAE-cellulose chromatography in Example 14, was subjected to electrophoresis on polyacrylamide gel at pH 6.0 to isolate a single substance. Dialyis and freeze drying gave 11mg of purified Protein D as white powder.

Example 16

Four grams of the crude cancer-cell killing and proliferation inhibiting factor obtained in the same manner as Example 12 were adsorbed on DEAE-cellulose (Whatman), and eluted with sodium chloride solution ( pH: 7.0 ) at a concentration gradient of from 0.03M to 0.01M to give four fractions.

The third fraction was dialyzed, adsorbed on DEAE-cellulose ( Whatman ), and eluted with Tris-HCl buffer solution ( pH: 7.0 ) at ion strength gradient from 0.03M to 0.05M to give four fractions.

The second fraction ( of the second DEAE-cellulose chromatography ) was dialyzed with 0.02M Tris-HCl buffer

solution, desalted and freeze-dried, affording 8.8mg of Protein E as white powder.

From the fourth fraction ( of the second DEAE-cellulose chromatography ) was obtained 13.7mg of Protein D as white powder after dialysis and freeze drying.

The Protein D and E            isolated above each exhibited a single band when submitted to electrophoresis.

These substances showed the same activities to inhibit the growth of cancer cells as Example 14.

Example 17

The second fraction ( 40mg ), obtained from the second DEAE-cellulose chromatography in Example 14, was subjected to electrophoresis on polyacrylamide gel at pH 6.0 to isolate the effective component as a single substance. Dialyis and freeze drying gave 6.8mg of purified Protein B as white powder.

Example 18

Tissue culture tests were conducted to investigate the inhibition of       the proliferation of various malignant tumour cells, for the Protein D  samples obtained in Examples 11,12 and 14 ( at 100 mcg/ml concentration ).

For leukemia L5178Y cells, the relative survival rate after five days was 10.1%, the relative activity to inhibit the cell growth being 79.5%.

For sarcoma S180 cells, the relative survival rate after five

0136093

-29-

days was 20.5%, the relative activity to inhibit the cell growth being 79.5%.

For Ehrlich's carcinoma cells, the relative survial rate after five days was 10.3%, the relative activity to inhibit the cell growth being 89.7%.

No such inhibitory effect was observed for the primary tissue-cultured cells of rat liver.

Example 19

Tissue culture tests were conducted to investigate the inhibition of the proliferation of various malignant tumour cells, for the Protein E samples obtained in Examples 14 and 16 ( at 100 mcg/ml concentration ).

For leukemia L5178Y cells, the relative survival rate after five days was 61%, the relative activity to inhibit the cell growth being 39%.

For sarcoma S180 cells, the relative survival rate after five days was 83%, the relative activity to inhibit the cell growth being 17%.

For Ehrlich's carcinoma cells, the relative survival rate after five days was 72%, the relative activity to inhibit the cell growth being 28%.

No such inhibitory effect was observed for the primary tissue-cultured cells of rat liver.

0136093

-30-

Example 20

An animal test was carried out using the purified Protein D  obtained in Example 15 from human clot to determine its effect upon ascites carcinoma.  Three groups of male BDF$_1$ mice ( weighing 20.g ) were used for the test, each group consisting of five head.

In the first group, 0.1mg of Protein A was abdominally administered to each mouse 24 hours after abdominal transplantation of leukemia L1210 cells ( 1 x 10$^6$ ).  Similarly, 0.01mg of the sample was administered in the second group.

The average life  time was 9.3 days for the control group, 21.7 days for the first group, and 15.7 days for the second, the T/C value being 233% and 169% for the first and second groups, respectively.

Example 21

An animal test was carried out using the purified Protein E  obtained in Example 17 from human clot to determine its effect upon ascites carcinoma.  Three groups of male BDF$_1$ mice ( weighing 20.g ) were used for the test, each group consisting of five head.

In the first group, 0.1mg of Protein E  was abdominally administered to each mouse 24 hours after abdominal transplantation of leukemia L1210 cells ( 1 x 10$^6$ ).  Similarly, 0.01mg of the sample was administered in the second group.

The average life time was 9.3 days for the control group, 14.7 days for the first group, and 13.0 days for the second, the T/C value being 158% and 140% for the first and second groups, respectively.

Example 22

An animal test was conducted using the Protein D obtained in Example 15 from human clot to determine its effect upon soild cancer.

Three groups of female ICR mice ( weighing 20g ) were used for the test, each group consisting of five head.

In the first group, 0.1mg of Protein D was hypodermically injected three times to each mouse —— one day, three days and ten days after hypodermal injection of sarcoma 180 cells ( $1 \times 10^7$ ) to the groin. The Protein D was similarly administered to the second group, except that the amount applied each time was 0.01mg.

In the control group, solid cancer averaging 4.5g in weight was observed after 30 days. On the contrary, the average weight of solid cancer developed in the first and second groups was 0.9g and 2.36g, respectively, the reduction rate being 80% and 48%.

Example 23

An animal test was conducted using the Protein E obtained in Example 17 from human clot to determine its effect upon soild cancer.

Three groups of female ICR mice ( weighing 20g ) were

-32-

used for the test, each group consisting of five head.

In the first group, 0.1mg of Protein E was hypodermically injected three times to each mouse —— one day, three days and ten days after hypodermal injection of sarcoma 180 cells ( $1 \times 10^7$ ) to the groin. The Protein E was similarly administered to the second group, except that the amount applied each time was 0.01mg.

In the control group, solid cancer averaging 4.5g in weight was observed after 30 days. On the contrary, the average weight of solid cancer developed in the first and second groups was 2.33g and 3.97g, respectively, the reduction rate being 48% and 12%.

-33-

The accompanying drawings, which are referred to above, are as follows:

Figure 1 is the infrared absorption spectrum of glycoprotein A of this invention;

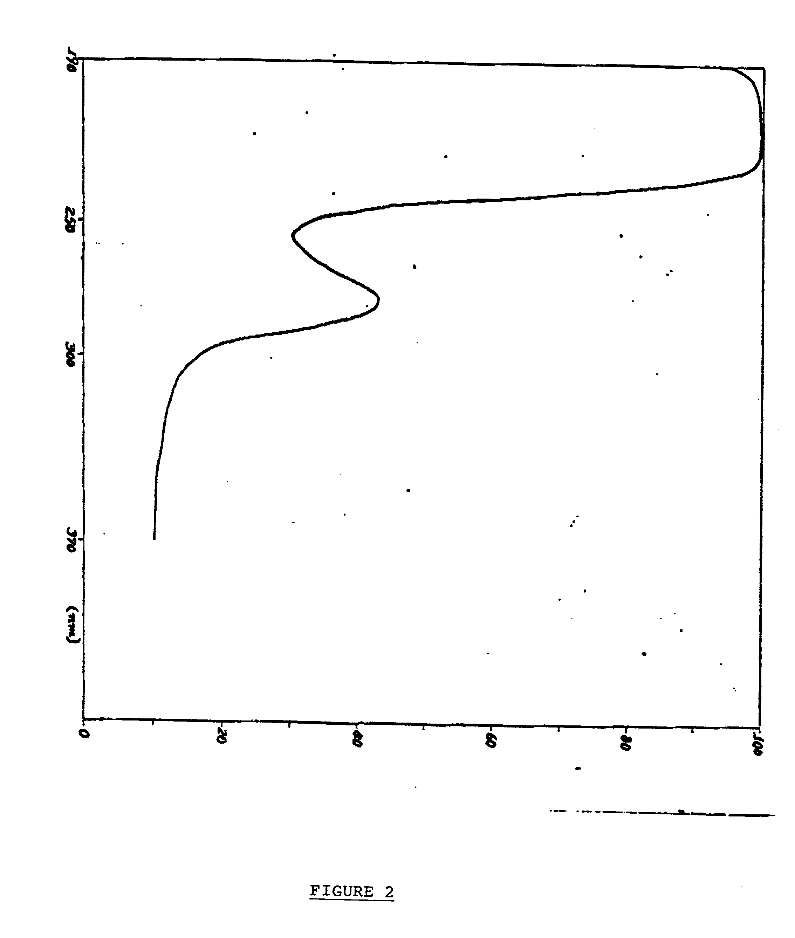

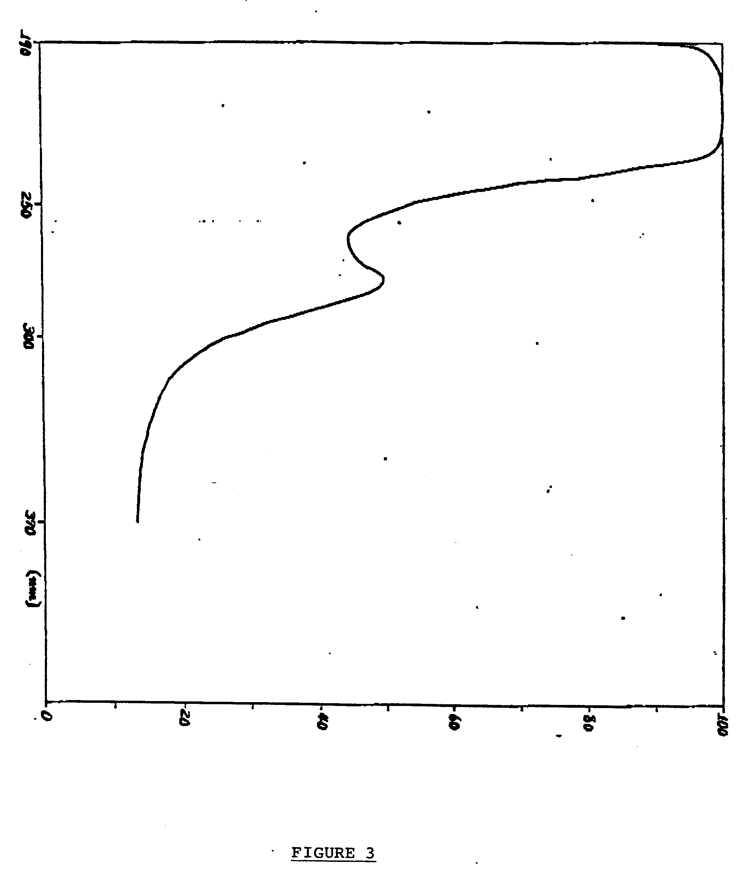

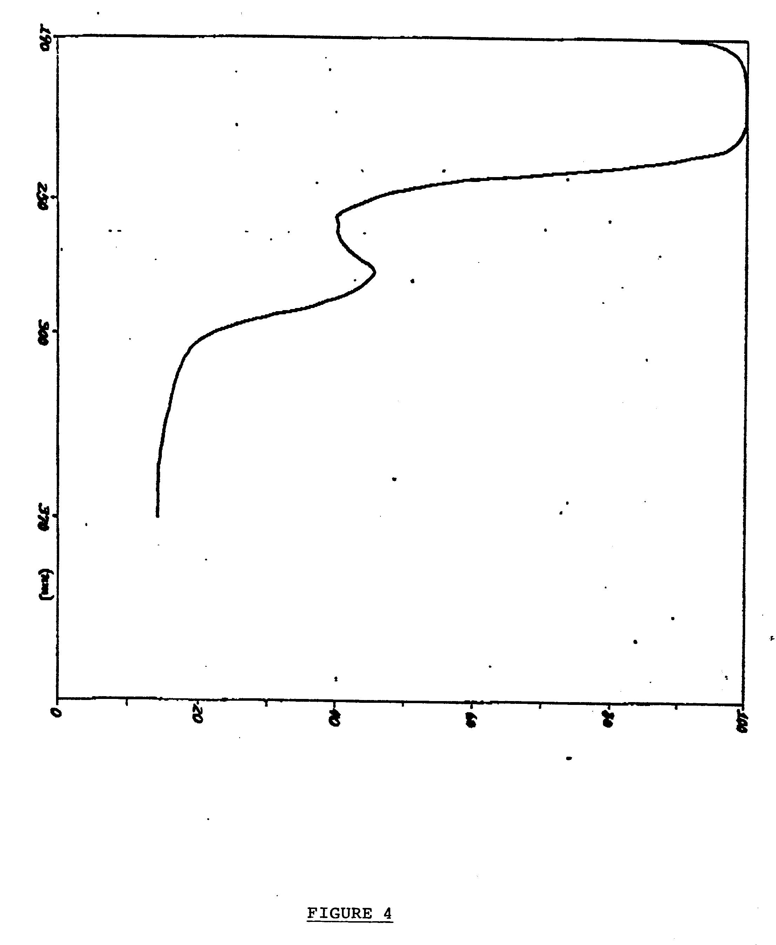

Figures 2, 3 and 4 show the ultraviolet absorption spectra of glycoprotein A at pH 3.0, 7.0 and 10.0, respectively;

Figure 5 is the infrared absorption spectrum of Protein B of this invention;

Figures 6, 7 and 8 show the ultraviolet absorption spectra of Protein B at pH 3.0, 7.0 and 10.0, respectively;

Figure 9 is the infrared absorption spectrum of Protein C of this invention;

Figures 10, 11 and 12 show the ultraviolet absorption spectrum of Protein C at pH 3.0, 7.0 and 10.0, respectively;

Figure 13 is the infrared absorption spectrum of Protein D of this invention;

Figure 14, 15 and 16 show the ultraviolet absorption spectra of Protein D at pH 5.0, 7.0 and 10.0, respectively;

Figure 17 is the infrared absorption spectrum of Protein E of this invention; and

Figures 18, 19 and 20 show the ultraviolet absorption spectra of Protein E at pH 5.0, 7.0 and 10.0, respectively.

0136093

-34-

CLAIMS:

1. A glycoprotein capable of being obtained from the human body, and designated A, characterised by:

(a) being a white powder;

(b) having a melting point in the range from 245°C to 249°C (with decomposition);

(c) having a molecular weight of approximately 73,900 a.m.u. when measured by electrophoresis in 10% sodium dodecylsulphate solution;

(d) being readily soluble in water, and insoluble in butanol, acetone, ethyl acetate, chloroform, benzene and hexane;

(e) having an amino acid composition of 10.0% aspartic acid, 5.4% threonine, 3.8% serine, 16.0% glutamic acid, 4.2% proline, 1.4% glycine, 4.8% alanine, 2.6% cysteine, 5.1% valine, 0.8£ methionine, 2.2% isoleucine, 9.6% leucine, 4.9% tyrosine, 5.7% phenylalanine, 11.4% lysine, 3.5% histidine and 5.2% arginine each in error of ± 10% to the % values;

(f) showing a positive response to the ninhydrine, anthrone, Molish and periodic acid reactions;

(g) giving an infrared absorption spectrum as shown in Figure 1 of the accompanying drawings (KBr pellet); and

(h) exhibiting ultraviolet absorption maxima, in water, at 279nm at pH 3.0, at 278nm at pH 7.0 and at 277nm at pH 10.0.

2. A protein capable of being obtained from the human body designated B, characterised by:

(a) being a white powder;

-35-

    (b)    having a melting point in the range from 221°C to 225°C (with decomposition);

    (c)    having a molecular weight of approximately 22,100 a.m.u. when measured by electrophoresis in 10% sodium doecylsulphate solution;

    (d)    being readily soluble in water, and insoluble in acetone, ethyl acetate, benzene, hexane and chloroform;

    (e)    having an amino acid composition of 16.0% aspartic acid, 5.2% threonine, 5.8% serine, 6.9% glutamic acid, 2.3% proline, 4.1% glycine, 5.9% alanine, 2.3% cysteine, 4.4% valine, 1.7% methionite, 4.2% isoleucine, 7.9% leucine, 4.0% tyrosine, 3.9% phenylalanine, 7.7% lysine, 1.6% histidine and 11.6% arginine; each in error of $\pm$ 10% to the % values;

    (f)    showing a positive response to the ninhydrine reaction and a negative response to the anthrone, Molish and periodic acid reactions;

    (g)    giving an infrared absorption spectrum as shown in Figure 5 of the accompanying drawings (KBr pellet); and

    (h)    exhibiting ultraviolet absorption maxima in water at 283nm at pH 3.0, at 280nm at pH 7.0 and at 277nm at pH 10.0.

3. A protein, capable of being obtained from the human body, designated C, characterised by:

    (a)    being a white powder;

    (b)    having a melting point in the range from 237°C to 240°C (with decomposition);

    (c)    having a molecular weight of approximately 14,000 a.m.u. when measured by electro-

0136093

-36-

phoresis in 10% sodium dodecylsulphate solution;

(d) being readily soluble in water, and insoluble in acetone, ethyl acetate, benzene, hexane and chloroform;

(e) having an amino acid compostion of 18.0% aspartic acid, 5.4% threonine, 6.4% serine, 5.2% glutamic acid, 1.7% proline, 5.1% glycine, 6.4% alanine, 2.1% cysteine, 4.3% valine, 1.9% methionine, 4.5 isoleucine, 6.8% leucine, 3.6% tyrosine, 3.0% phenylalanine, 6.0% lysine, 1.1% histidine and 11.6% arginine, each in error of $\pm$ 10% to the % values;

(f) showing a positive response to the ninhydrine reaction and a negative response to the anthrone, Molish and periodic acid reactions;

(g) giving an infrared absorption spectrum as shown in Figure 9 of the accompanying drawings (KBr pellet); and

(h) exhibiting ultraviolet absorption maxima in water at 278nm at pH 3.0, at 290 nm at 7.0, and at 277nm at pH 10.0.

4. A composition which comprises two or three of the proteins A, B and C as claimed in Claims 1, 2 and 3, respectively.

5. A protein capable of being obtained from the human body and designated D, characterised by:

(a) being a white powder;

(b) melting in the range from 218°C to 221°C (with decomposition);

(c) having a molecular weight of approximately 40,000 a.m.u. when measured by

-37-

electrophoresis in 10% sodium
dodecylsulphate solution;

(d)    being readily soluble in water, and
       insoluble in butanol, acetone, ethyl
       acetate, chloroform, benzene and hexane;

(e)    having an amino acid composition of
       8.9% aspartic acid, 5.7% threonine,
       4.3% serine, 6.3% glutamic acid, 3.8%
       proline, 5.8% glycine, 5.8% alanine,
       0.6% cysteine, 5.2% valine, 0.5% methionine,
       2.2% isoleucine, 4.7% leucine, 3.0%
       tyrosine, 2.6% phenylalanine, 2.4%
       lysine, 1.3% histidine and 4.4% **arginine;**
       each in error of $\pm$ 10% to the % values;

(f)    showing a positive respone to the
       ninhydrine reaction and a negative
       response to the anthrone, Molish and
       periodic acid reactions;

(g)    giving an infrared absorption spectrum
       as shown in Figure 13 of the accompanying
       drawings (KBr pellet); and

(h)    exhibiting ultraviolet absorption
       in water at 275nm, 282.5nm and 291.5nm
       at pH 5.0, at 275nm, 282.5nm and 291.5nm
       at pH 7.0, and at 282nm and 291.5nm
       at pH 10.0.

6.    A protein, capable of being obtained
from the human body,     designated E,
characterised by:

(a)    being a white powder;

(b)    having a melting point in the range
       from 231°C to 233°C (with decomposition);

(c)    having a moleuclar weight of approximately
       44,000 a.m.u. when measured by
       electrophoresis in 10% sodium
       dodecylsulphate solution;

(d)    being readily soluble in water, and

0136093

-38-

insoluble in acetone, ethyl acetate, chloroform, benzene and hexane;

(e) having an amino acid composition of 9.2% aspartic acid, 5.7% threonine, 4.3% serine, 8.6% glutamic acid, 4.0% proline, 4.7% glycine, 5.6% alanine, 1.0% cysteine, 5.2% valine, 0.6% methionine, 2.2% isoleucine, 6.0% leucine, 3.4% tyrosine, 3.1% phenylalanine, 4.4% lysine, 1.9% histidine and 4.7% **arginine**; each in error of $\pm$ 10% to the % values;

(f) showing a positive response to the ninhydrine reaction and a negative response to the anthrone, Molish and periodic acid reactions;

(g) giving an infrared absorption spectrum as shown in Figure 17 of the accompanying drawings (KBr pellet); and

(h) exhibiting ultraviolet absorption maxima in water at 280nm (with a shoulder at 249nm) at pH 5.0, at 280nm (with a shoulder at 291nm) at pH 7.0, and at 281nm (with a shoulder at 293nm) at pH 10.0.

7. A composition which comprises the Proteins D and E as claimed in Claims 5 and 6 respectively.

8. A process for preparing a compound as claimed in any one of Claims 1, 2, 3, 5 and 6, which process comprises separating the desired compound from a sample of human blood or human serum.

9. A process according to Claim 8, wherein the treated product is purified.

FIGURE 1

FIGURE 2

FIGURE 3

0136093

FIGURE 4

0136093

FIGURE 5

0136093

FIGURE 6

0136093

FIGURE 7

0136093

FIGURE 8

0136093

FIGURE 9

0136093

FIGURE 10

0136093

11/20

FIGURE 11

FIGURE 12

FIGURE 13

FIGURE 14

15°/2c

FIGURE 15

**FIGURE 16**

FIGURE 17

0136093

FIGURE 18

FIGURE 19

FIGURE 20